# EUROPEAN PATENT APPLICATION

(11) **EP 4 316 663 A1**
(43) Date of publication of application: **07.02.2024**
(21) Application number: 23186993.4
(22) Date of filing: 21.07.2023
(51) Int. Cl.: B01L 3/02, B04B 5/04, G01N 35/10, B01L 3/14

(54) **LIQUID SUCTION DEVICE**

(30) Priority: 01.08.2022 JP 2022123016
(71) Applicant: Shibuya Corporation, Kanazawa-shi, Ishikawa 920-8681 (JP)
(72) Inventor: DEGUCHI, Naoya, Kanazawa-shi, 920-8681 (JP); YAMASHITA, Takahiro, Kanazawa-shi, 920-8681 (JP); NISHITA, Takuto, Kanazawa-shi, 920-8681 (JP)
(74) Representative: Mewburn Ellis LLP

(57) **Abstract**

Only a supernatant in a centrifugal separation container is drawn out and removed as much as possible. The present disclosure relates to a liquid suction device 1 including container holding means 3 that holds a centrifugal separation container 2 in which a mouth portion 2d is formed at an upper portion, and a tubular main housing portion 2a and a conical bottom housing portion 2b below the main housing portion 2a are formed inside, with the bottom housing portion 2b facing down; a cylindrical suction nozzle N that is inserted into the centrifugal separation container 2; and suction means 8 to which the suction nozzle N is connected to draw out a liquid in the centrifugal separation container 2. Container inclining means 4 that inclines the container holding means 3, and nozzle inserting means 6 that inserts the suction nozzle N into an inside of the centrifugal separation container 2 are included, the container inclining means 4 inclines the container holding means 3 so that a boundary portion 2c between the main housing portion 2a and the bottom housing portion 2b in an inner peripheral surface of the centrifugal separation container 2 is at a lowermost end position, and a supernatant L is drawn out by the suction means in a state in which a tip end of the suction nozzle N is positioned at the boundary portion 2c at the lowermost end position.

## Description

### BACKGROUND OF THE INVENTION

### Field of the Invention

The present invention relates to a liquid suction device, and more particularly relates to a liquid suction device that draws out a liquid from a centrifugal separation container in which a conical bottom housing portion is formed.

### Description of the Related Art

Conventionally, when collecting cultured cells, a cell suspension that contains cells and is housed in a centrifugal separation container is treated by a centrifugal separation device, the cell suspension is separated into cells and a supernatant, and only the supernatant is removed from the centrifugal separation container.

The centrifugal separation container that is used to separate the cells by the above-described centrifugal separation device generally has a mouth portion formed at an upper portion, and includes a tubular main housing portion and a conical bottom housing portion below the main housing portion, inside.

When the cell suspension is centrifuged by using the centrifugal separation container, the separated cells are pushed into a conical tip end portion of the bottom housing portion, and the supernatant accumulates in an upper portion thereof.

Operations of removing the supernatant from the centrifugal separation container have been performed manually by using pipets and aspirators, but in recent years, there has been a demand for automating these operations.

As such an automated device, there is known a configuration in which a suction nozzle is inserted into a centrifugal separation container held by a centrifugal separation device to draw out a supernatant (Japanese Laid-Open Patent Application No. 2011-4705).

Here, in the liquid suction device of Japanese Laid-Open Patent Application No. 2011-4705, a tip end portion 11a of a suction nozzle 11 (see FIG. 2) for removing the supernatant is positioned above the cells in the bottom housing portion of the centrifugal separation container to draw out the supernatant accumulated above the tip end portion 11a.

In the device of Japanese Laid-Open Patent Application No. 2011-4705 described above, the position of the tip end of the suction nozzle is set to be spaced upward from the cells to allow a margin, so as not to draw out cells by mistake, since the amount of cells differs from one centrifugal separation container to another.

Consequently, there has been a problem that while the supernatant is drawn out by the suction nozzle, the supernatant accumulated between the upper surfaces of the cells and the tip end of the suction nozzle cannot be removed.

In view of the problem as above, the present invention provides a liquid suction device that can collect only a supernatant as much as possible from a centrifugal separation container.

### SUMMARY OF THE INVENTION

A liquid suction device according to the invention of claim 1 is a liquid suction device comprising a container holding means that holds a centrifugal separation container in which a mouth portion is formed at an upper portion, and a tubular main housing portion and a conical bottom housing portion below the main housing portion are formed inside, with the bottom housing portion facing down; a cylindrical suction nozzle that is inserted into the centrifugal separation container; and a suction means to which the suction nozzle is connected to draw out a liquid in the centrifugal separation container, the liquid suction device being characterized by comprising:
the container inclining means that inclines the container holding means; and nozzle inserting means that inserts the suction nozzle into an inside of the centrifugal separation container, and characterized in that
the container inclining means inclines the container holding means so that a boundary portion between the main housing portion and the bottom housing portion in an inner peripheral surface of the centrifugal separation container is at a lowermost end position, and the liquid is drawn out by the suction means in a state in which a tip end of the suction nozzle is positioned at the boundary portion at the lowermost end position.

According to the above-described invention, by inclining the centrifugal separation container held by the container holding means with the container inclining means, the boundary portion between the main housing portion and the bottom housing portion can be positioned at the lowermost end position in the centrifugal separation container. Since at this time, the cells after centrifugal separation maintain the state in which the cells are pushed in and fit into the tip end portion of the conical bottom housing portion, only the supernatant collects at the boundary portion at the lowermost end position when the centrifugal separation container is inclined.

Then, by drawing out the liquid in the state in which the tip end of the suction nozzle is positioned at the boundary portion at the lowermost end position, it is possible to draw out and remove only the supernatant as much as possible without drawing out the cells.

### BRIEF DESCRIPTION OF THE DRAWINGS

FIG. 1 is a front view of a liquid suction device in a first embodiment;
FIG. 2 is a side view of the liquid suction device in the first embodiment;
FIGS. 3(a), 3(b), and 3(c) are views each explaining operation of the liquid suction device;
FIGS. 4(a), 4(b), and 4(c) are views each showing a positional relationship between a centrifugal separation container and a suction nozzle;
FIG. 5 is a front view of a liquid suction device in a second embodiment;
FIG. 6 is an operation view of the liquid suction device in the second embodiment;
FIG. 7 is a front view of a liquid suction device in a third embodiment; and
FIG. 8 is an operation view of the liquid suction device in the third embodiment.

### DETAILED DESCRIPTION OF THE PREFERRED EMBODIMENTS

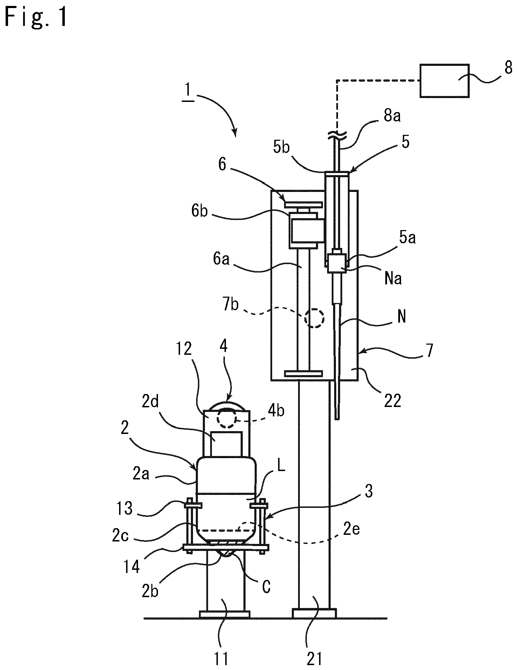

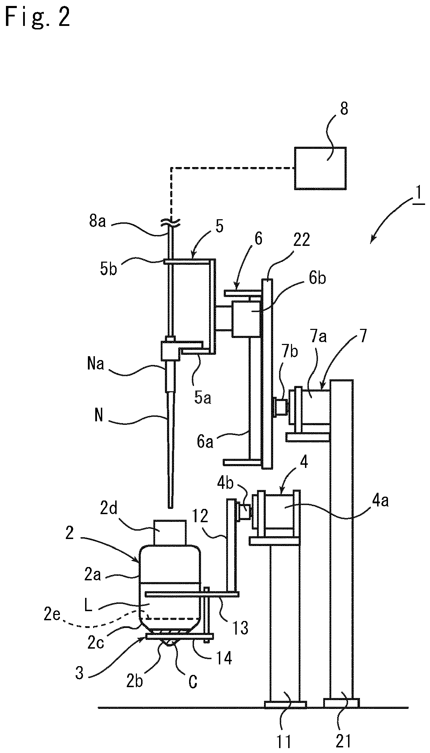

Describing illustrated embodiments hereinafter, FIG. 1 and FIG. 2 show a front view and a side view of a liquid suction device 1 according to a first embodiment, which draws out a liquid from a centrifugal separation container 2 for being used in a centrifugal separation device.

The liquid suction device 1 is provided inside of an isolator configuring a cell culture system an inside of which is kept in an aseptic condition, and the centrifugal separation device, a nozzle replacing device for replacing a suction nozzle described later, and the like are provided inside of the isolator. Further, the cell culture system is controlled by control means including a computer and the like not illustrated, and each step for performing cell culture is automatically processed.

Though detailed explanation of the operation of the above-described cell culture system is omitted, the liquid suction device 1 of the present embodiment is used in culture medium exchange and cell collection that are performed in a process of cell culture.

On the medium exchange and cell collection, a cell suspension containing cells and a culture solution is collected from a culture vessel and housed in the centrifugal separation container 2, and the cell suspension housed in the centrifugal separation container 2 is centrifuged in a centrifugal separation device.

By centrifugal separation, the cell suspension is separated into cells C, and a supernatant L containing a culture solution and the like, and of them, the cells C are pushed in and fit into a tip end portion of a conical bottom housing portion 2b of the centrifugal separation container 2 by a centrifugal force, and the supernatant L accumulates above the cells C in a liquid state.

In order to collect only the cells C from the centrifugal separation container 2, only the supernatant L is drawn out and removed from the centrifugal separation container 2 by using the liquid suction device 1 of the present embodiment.

A conventionally known centrifugal separation container can be used as the centrifugal separation container 2 used in the present embodiment, the centrifugal separation container 2 includes a tubular main housing portion 2a and the conical bottom housing portion 2b formed below the main housing portion 2a inside, a boundary portion 2c having a smooth arc-shaped cross-section is provided in an inner peripheral surface between the main housing portion 2a and the bottom housing portion 2b.

A mouth portion 2d having a smaller diameter than that of the main housing portion 2a and a cap not illustrated fitted thereon is formed on an upper portion of the main housing portion 2a. Note that the main housing portion 2a may have a tapered shape that decreases in diameter toward the bottom housing portion 2b.

Here, since an amount of the cells C centrifuged by the centrifugal separation device differs at each time, a position (height) of an upper surface of the cells C in the centrifugal separation container 2 varies, and in the present embodiment, the height of the upper surface of the cells C is positioned in a range of a conical shape portion configuring the bottom housing portion 2b so that only the supernatant L flows into the boundary portion 2c that is at a lowermost end position, so as not to be affected by the amount of cells C during suction.

The liquid suction device 1 includes container holding means 3 that holds the centrifugal separation container 2, container inclining means 4 that inclines the container holding means 3, a cylindrical suction nozzle N that is inserted into the centrifugal separation container 2, nozzle holding means 5 that holds the suction nozzle N, nozzle inserting means 6 that supports the nozzle holding means 5 to insert the suction nozzle N into an inside of the centrifugal separation container 2, nozzle inclining means 7 that inclines the nozzle inserting means 6, and suction means 8 connected to the suction nozzle N.

The container holding means 3 is supported by a first strut 11 fixed to a floor surface of the isolator via the container inclining means 4, is configured by a support member 13 that is provided at a back plate 12 included by the container inclining means 4 to support the centrifugal separation container 2 from sides, and a support plate 14 provided by being suspended from the support member 13 and has a through-hole (not illustrated) in which the bottom housing portion 2b of the centrifugal separation container 2 is fitted, formed substantially in a center, and holds the centrifugal separation container 2 with the bottom housing portion 2b facing down.

In the present embodiment, it is possible to perform feeding and unloading of the centrifugal separation container 2 to and from the container holding means 3 by transfer means such as a robot or the like not illustrated, and in order to avoid interference, the suction nozzle N and the nozzle holding means 5 are not positioned above the container holding means 3 as shown in FIG. 1.

The container inclining means 4 is configured by a motor 4a provided at an upper end portion of the first strut 11 as shown in FIG. 2, and a driving shaft 4b of the motor 4a is coupled to the back plate 12.

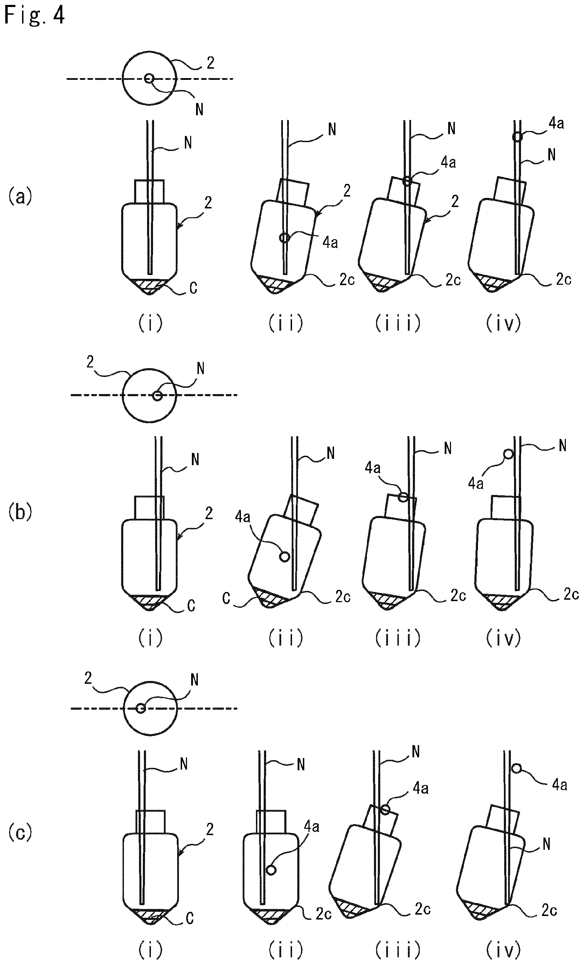

As will be described later by using FIGS. 4(a), 4(b), and 4(c), a position of the driving shaft 4b is considered to be set at a higher position than an upper end of the mouth portion 2d of the centrifugal separation container 2, a position at an equal height to a height of the upper end of the mouth portion 2d, or a lower position than the upper end of the mouth portion 2d, and in the present embodiment, the driving shaft 4b is set at the position at the equal height to the height of the upper end of the mouth portion 2d of the centrifugal separation container 2.

The suction nozzle N is a long and narrow cylindrical member, is formed into a tapered shape tapering toward a tip end, and has an upper end portion joined to a connection tube Na supported by the nozzle holding means 5.

In the present embodiment, the suction nozzle N is replaceable, and nozzle replacing means not illustrated holds the suction nozzle N to attach and detach the suction nozzle N to and from the connection tube Na. The nozzle holding means 5 is provided at a second strut 21 provided in the isolator via the nozzle inserting means 6 and nozzle inclining means 7, and includes a coupling portion 5a to which the connection tube Na is coupled, and a tube holding portion 5b that is provided above the coupling portion 5a and holds a flexible tube 8a connected between the suction nozzle N and suction means 8.

As shown in FIG. 1, the second strut 21 is provided to be displaced in a direction (rightward in the present embodiment) in which the container holding means 3 (centrifugal separation container 2) and the nozzle inserting means 6 (suction nozzle N) are inclined, with respect to the first strut 11 that holds the container holding means 3, so that in a state in which the centrifugal separation container 2 and suction nozzle N stand upright, the centrifugal separation container 2 is not positioned below the suction nozzle N.

This can prevent the suction nozzle N from interfering with the centrifugal separation container 2 when the suction nozzle N is replaced by the nozzle replacing means, and can prevent foreign matter from falling inside the centrifugal separation container 2 during replacement.

The nozzle inserting means 6 is composed of a linear slider, and configured by a rod 6a provided on a front surface of a back plate 22 inclined by the nozzle inclining means 7, and a slider 6b that raises and lowers the suction nozzle N together with the nozzle holding means 5 along the rod 6a.

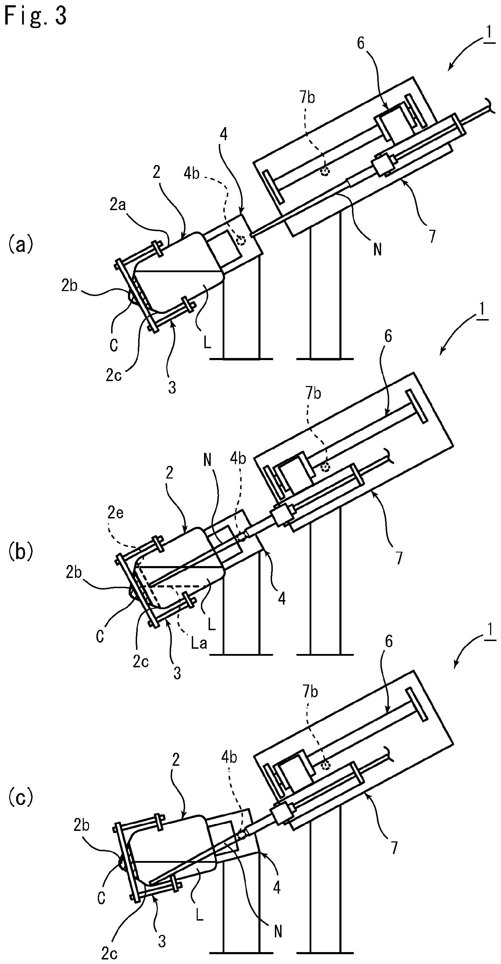

The slider 6b is driven by driving means not illustrated, and moves along the rod 6a, whereby the suction nozzle N held by the nozzle holding means 5 moves to be inserted into the centrifugal separation container 2, and it becomes possible to insert the suction nozzle N into the centrifugal separation container 2 as shown in FIGS. 3(a), 3(b) and 3 (c) .

The nozzle inclining means 7 is configured by a motor 7a provided at an upper end portion of the second strut 21 as shown in FIG. 2, and a driving shaft 7b of the motor 7a is coupled to the back plate 22.

In the present embodiment, as shown in FIGS. 3(a) and 3(b), the container inclining means 4 and nozzle inclining means 7 incline the container holding means 3 and the centrifugal separation container 2, and the nozzle inserting means 6 and the suction nozzle N in the same direction.

Further, in the present embodiment, as shown in FIGS. 3(a) and 3(b), the centrifugal separation container 2 and the suction nozzle N are inclined to the same angle, and at this time, a center axis of the suction nozzle N and a center axis (center position of the mouth portion 2d) of the centrifugal separation container 2 are caused to coincide with each other.

However, concerning a positional relationship between the center axis of the suction nozzle N and the center position of the mouth portion 2d of the centrifugal separation container 2, the suction nozzle N can also be set to a position eccentric in an opposite direction (leftward in the drawing) to an inclining direction with respect to the mouth portion 2d as will be described later by using FIGS. 4(a), 4(b), and 4(c).

An operation of the liquid suction device 1 having the configuration will be described. First, in the centrifugal separation device, centrifugal separation treatment is performed for the centrifugal separation container 2 in which the cell suspension is housed.

Thereby, the cell suspension is centrifuged, the cells C are pushed in and fit into the tip end portion of the bottom housing portion 2b of the centrifugal separation container 2, and the liquid supernatant L accumulates above the cells C.

The centrifugal separation container 2 after the centrifugal separation in which the cells C and supernatant L are housed is transferred to the container holding means 3 in the liquid suction device 1 manually or the transfer device such as a robot, the nozzle replacing means not illustrated moves the unused suction nozzle N to the nozzle holding means 5 to join the unused suction nozzle N to the connection tube Na, and connects the tube 8a of the suction means 8 to the suction nozzle N.

Since at this time, the container holding means 3 and the nozzle inserting means 6 are in an upright state as shown in FIG. 1 and FIG. 2, and the nozzle holding means 5 and the suction nozzle N are not positioned above the container holding means 3, it is possible to easily perform transfer of the centrifugal separation container 2. Similarly to this, the centrifugal separation container 2 is not positioned below the nozzle holding means 5, and therefore it is possible to easily perform replacement of the suction nozzle N.

When the centrifugal separation container 2 and the suction nozzle N are set in this way, the control means operates the container inclining means 4 and nozzle inclining means 7 to incline the container holding means 3 and the nozzle inserting means 6 by the same angle in the same direction as shown in FIG. 3(a).

Thereby, the center axis of the centrifugal separation container 2 and the center axis of the suction nozzle N are positioned on the same axis. Further, the boundary portion 2c of the main housing portion 2a and the bottom housing portion 2b in the inner peripheral surface of the centrifugal separation container 2 is at the lowermost end position of the centrifugal separation container 2.

Here, when the centrifugal separation container 2 is inclined by the container inclining means 4, the supernatant L moves so that a liquid surface is kept horizontal since the supernatant L is a liquid, but the cells C maintain the state in which the cells C are pushed in and fit into the conical bottom housing portion 2b of the centrifugal separation container 2, so that a shape of the integrated cells C does not change and does not collapse to the boundary portion 2c.

Next, the control means operates the nozzle inserting means 6 to move the suction nozzle N toward the centrifugal separation container 2 and inserts the suction nozzle N into the centrifugal separation container 2 as shown in FIG. 3(b).

At this time, the suction nozzle N is inserted into a vicinity of a lower end 2e of the main housing portion 2a inside of the centrifugal separation container 2, and is stopped at a position separated from the top surface of the cells C fitted into the bottom housing portion 2b.

In this state, the tip end of the suction nozzle N is not directed to the boundary portion 2c of the centrifugal separation container 2 since the center axis of the centrifugal separation container 2 and the center axis of the suction nozzle N are positioned on the same axis, but if the centrifugal separation container 2 is inclined any further, the supernatant L flows out from the mouth portion 2d, and therefore, in the state in which the inclination angle is maintained temporarily, the suction means 8 is operated to draw out the supernatant L.

However, in this state, the supernatant L below a position of the tip end of the suction nozzle N (below a broken line La illustrated in FIG. 3(b)) cannot be drawn out.

If the treatment is finished in the state in which the supernatant L remains in this way, the supernatant L will be mixed in when the cells C are collected from the centrifugal separation container 2 thereafter, so that it is necessary to remove as much of the supernatant L as possible.

Thus, in the present embodiment, as shown in FIG. 3(c), the container inclining means 4 further inclines the container holding means 3 in the direction in which the container holding means 3 has already been inclined.

Thereby, the suction nozzle N is relatively inclined inside of the centrifugal separation container 2, and the tip end of the suction nozzle N is brought into a state where it is positioned at the boundary portion 2c of the main housing portion 2a and the bottom housing portion 2b, in the inner peripheral surface of the centrifugal separation container 2.

In this state, the boundary portion 2c is still at the lowermost end position of the centrifugal separation container 2, and it is possible to draw out an entire amount of the remaining supernatant L by maintaining the container holding means 3 (centrifugal separation container 2) at this inclination angle and operating the suction means 8.

Note that also in this state, the cells C maintain the state fitted into the bottom housing portion 2b, and therefore, the cells C do not collapse to the boundary portion 2c.

Here, the inclination angle of the container holding means 3 and the centrifugal separation container 2 by the container inclining means 4, an insertion amount of the suction nozzle N to the centrifugal separation container 2 by the nozzle inserting means 6, and the inclination angle of the nozzle inserting means 6 and the suction nozzle N by the nozzle inclining means 7 are registered in control means not illustrated, in advance.

Note that depending on a housed amount of the supernatant L of the centrifugal separation container 2, after the container holding means 3 and nozzle inserting means 6 are inclined in the same direction, only the container holding means 3 may be further inclined continuously, and thereafter, the nozzle inserting means 6 may advance the suction nozzle N to insert the suction nozzle N into the centrifugal separation container 2, and may position the tip end of the suction nozzle N at the boundary portion 2c.

Since the boundary portion 2c in the inner peripheral surface of the centrifugal separation container 2 is at the lowermost end position of the centrifugal separation container 2 by the container inclining means 4 in this way, the supernatant L collects at the boundary portion 2c, and by positioning the tip end of the suction nozzle N at the boundary portion 2c and applying suction, it is possible to remove substantially the entire amount of the supernatant L remaining in the centrifugal separation container 2.

Note that when the tip end of the suction nozzle N is positioned at the boundary portion 2c, the suction nozzle N may be inclined in the same direction as the centrifugal separation container 2 shown in FIG. 3(a) by the nozzle inclining means 7 without further inclining the centrifugal separation container 2, and thereafter, the suction nozzle N may be returned in an opposite direction by a predetermined angle.

In other words, by inclining the suction nozzle N vertically inserted into the centrifugal separation container 2 with mutual center axes coinciding with each other by a predetermined amount relatively to the centrifugal separation container 2, it is possible to position the tip end of the suction nozzle N at the boundary portion 2c in the inner peripheral surface of the centrifugal separation container 2.

After the supernatant L in the centrifugal separation container 2 is removed in this way, the control means operates and inserts the container inclining means 4, the nozzle inserting means 6, and the nozzle inclining means 7 in a reverse order from the previous order to withdraw the suction nozzle N from the centrifugal separation container 2, and stand the centrifugal separation container 2 and the suction nozzle N upright.

Here, FIGS. 4(a), 4(b), and 4(c) are views in which the insertion states of the suction nozzle N are compared when the positional relationship of the center axis of the centrifugal separation container 2 and the center axis of the suction nozzle N, and the position of the driving shaft 4a of the container inclining means 4 are respectively made to differ.

Note that here, the centrifugal separation container 2 is not inclined so that the boundary portion 2c is positioned at the lowermost end position of the centrifugal separation container 2.

FIG. 4(a) shows a case in which the center axis of the centrifugal separation container 2 and the center axis of the suction nozzle N are caused to coincide with each other.

In contrast to this, FIG. 4(b) shows a case in which the center axis of the suction nozzle N is made eccentric in the direction in which the centrifugal separation container 2 is inclined with respect to the center axis of the centrifugal separation container 2 (deviated rightward illustrated in FIG. 1), and FIG. 4(c) shows a case in which the center axis of the suction nozzle N is made eccentric in the opposite direction to the direction in which the centrifugal separation container 2 is inclined, with respect to the center axis of the centrifugal separation container 2 (deviated leftward illustrated in FIG. 1).

Further, in each of FIGS. 4(a) to 4(c), (i) shows a state in which the suction nozzle N is inserted without being inclined with respect to the centrifugal separation container 2.

In contrast to this, (ii), (iii) and (iv) show states in which the height positions of the driving shaft 4a of the container inclining means 4 are made to differ when the suction nozzle N is relatively inclined with respect to the centrifugal separation container 2 so that the tip end is positioned at the boundary portion 2c, (ii) shows a case in which the driving shaft 4a is positioned at the position lower than the upper end of the mouth portion 2d of the centrifugal separation container 2, (iii) shows a case in which the driving shaft 4a is positioned at the position at the equal height to the height of the upper end of the mouth portion 2d of the centrifugal separation container 2, and (iv) shows a case in which the driving shaft 4a is positioned at the position higher than the upper end of the mouth portion 2d of the centrifugal separation container 2, respectively.

FIG. 4(a) shows the case in which the center axis of the centrifugal separation container 2 and the center axis of the suction nozzle N coincide with each other, as in the present embodiment.

In this state, if the centrifugal separation container 2 is inclined as shown in (ii), the mouth portion 2d of the inclined centrifugal separation container 2 interferes with the suction nozzle N, and the tip end of the suction nozzle N cannot be positioned at the boundary portion 2c.

In contrast to this, if the centrifugal separation container 2 is inclined as shown in (iii) and (iv), it becomes possible to position the tip end of the suction nozzle N at the boundary portion 2c.

In FIG. 4(b), the tip end of the suction nozzle N can be positioned at the boundary portion 2c when the driving shaft 4a is at the same height as the height of the upper end of the mouth portion 2d as shown in (iii). When the driving shaft 4a is at the position lower than the upper end of the mouth portion 2d as shown in (ii), the tip end of the suction nozzle N can be directed to the boundary portion 2c but cannot be positioned at the boundary portion 2c. When the driving shaft 4a is at the position higher than the upper end of the mouth portion 2d as shown in (iv), the tip end of the suction nozzle N cannot be positioned at the boundary portion 2c.

In FIG. 4(c), it is possible to position the tip end of the suction nozzle N at the boundary portion 2c, when the driving shaft 4a is at the same height as the height of the upper end of the mouth portion 2d as shown in (iii), or when the driving shaft 4a is at the position higher than the upper end of the mouth portion 2d as shown in (iv), and when the driving shaft 4a is at the position lower than the upper end of the mouth portion 2d as shown in (ii), it is not even possible to incline the suction nozzle N.

From the above, it is found that as the position in the height direction of the driving shaft 4a to the centrifugal separation container 2, that is, the center of rotation for inclining the centrifugal separation container 2, the position at the height substantially equal to the height of the upper end of the mouth portion 2d of the centrifugal separation container 2, or the position higher than the upper end of the mouth portion 2d is suitable. Further, as the position of the suction nozzle N to the center axis of the centrifugal separation container 2, the position of the center axis, or the position eccentric in the opposite direction to the inclining direction from the position of the center axis is suitable.

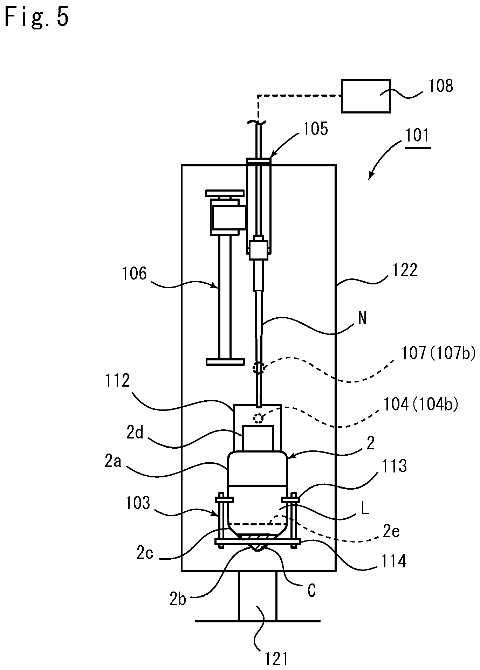

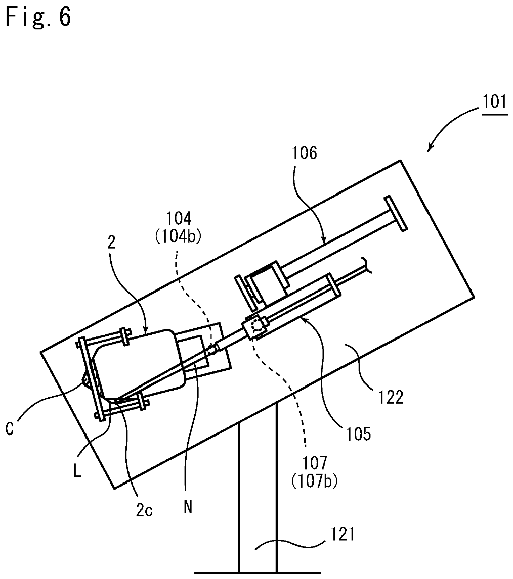

FIG. 5 and FIG. 6 show a liquid suction device 101 according to a second embodiment. Note that in the following explanation, detailed explanation of the same components as those of the first embodiment will be omitted, and numbers that are obtained by adding 100 to the reference signs used in the first embodiment will be assigned to them.

While in the first embodiment, the container inclining means 4 and nozzle inclining means 7 are respectively provided at the first strut 11 and the second strut 21, in the present embodiment, container holding means 103 and container inclining means 104 are provided at the nozzle inclining means 7 to configure inclining operation means 107, and thereby the container holding means 103 (centrifugal separation container 2) and nozzle inserting means 106 (suction nozzle N) are configured to be integrally inclined in the same direction.

The inclining operation means 107 is configured by a motor 107a not illustrated and provided at an upper portion of a strut 121, and a back plate 122 is coupled to a driving shaft 107b of the motor 107a.

On a front surface of the back plate 122, the nozzle inserting means 106 is provided, and nozzle holding means 105 is supported by the nozzle inserting means 106. As shown in FIG. 5, the container holding means 103 is disposed below the nozzle holding means 105 and the suction nozzle N such that the centrifugal separation container 2 is positioned.

The container holding means 103 and container inclining means 104 are provided on the back plate 122, a motor 104a not illustrated is provided on the front surface of the back plate 122, and a support member 113 and a support plate 114 that configure the container holding means 103 are provided at the back plate 112 coupled to a driving shaft 104b of the motor 104a.

Note that the driving shaft 104b is provided at a position slightly higher than an upper end of a mouth portion 2d of the centrifugal separation container 2 held by the container holding means 103, as in the first embodiment.

With the configuration, in a state in which the container inclining means 104 causes the container holding means 103 and the centrifugal separation container 2 to stand upright as shown in FIG. 5, a center axis of the centrifugal separation container 2 and a center axis of the suction nozzle N held by the nozzle holding means 105 coincide with each other.

An operation of the liquid suction device 101 according to the second embodiment having the configuration will be described.

The inclining operation means 107 brings the nozzle inserting means 106 into an upright state and causes the nozzle holding means 105 to hold the suction nozzle N upright. In this state, the container holding means 103 is caused to hold the centrifugal separation container 2 housing cells C and a supernatant L, and the centrifugal separation container 2 is positioned directly under the suction nozzle N.

When the liquid suction device 101 is operated from this state, the nozzle inserting means 106 inserts the suction nozzle N into the centrifugal separation container 2 first. Since the centrifugal separation container 2 and the suction nozzle N both stand upright at this time, the suction nozzle N passes through a center of the centrifugal separation container 2 to reach a vicinity of a lower end 2e of a main housing portion 2a, and is positioned above the cells C fitted into a bottom housing portion 2b.

Suction means 108 is temporarily operated in this state to draw out the supernatant L up to the vicinity of the lower end 2e of the main housing portion 2a. In this state, the supernatant L remains above the cells C in the bottom housing portion 2b of the centrifugal separation container 2.

Subsequently, the inclining operation means 107 is operated by control means not illustrated to incline the nozzle inserting means 106 and suction nozzle N and integrally incline the container holding means 103 and the centrifugal separation container 2 together with the container inclining means 104.

At this time, relative inclination angles of the centrifugal separation container 2 and the suction nozzle N are maintained at the same angle, and therefore, the centrifugal separation container 2 and the suction nozzle N that are inclined are in a similar state to the state in FIG. 3(b) shown in the above-described first embodiment.

Continuously from this state, the container inclining means 104 is operated by the control means not illustrated to incline only the container holding means 103 further in the same direction as shown in FIG. 6 to an inclination angle different from the inclination angle of the nozzle inserting means 106, and a tip end of the suction nozzle N is positioned at a boundary portion 2c between the main housing portion 2a and the bottom housing portion 2b in an inner peripheral surface of the centrifugal separation container 2.

In this state, the boundary portion 2c of the centrifugal separation container 2 is at a lowermost end position, and by operating the suction means 108 while maintaining this state, it is possible to draw out substantially the entire amount of the supernatant L remaining in the centrifugal separation container 2, as in the first embodiment.

Note that in the present second embodiment, the inclining operation means 107 inclines the container holding means 103 so that the boundary portion 2c in the inner peripheral surface of the centrifugal separation container 2 is at the lowermost end position, and is configured as container inclining means in the present invention.

Here, in the above-described second embodiment, by further inclining only the container holding means 103 by the container inclining means 104, the tip end of the suction nozzle N is positioned at the boundary portion 2c that is at the lowermost end position.

In contrast to this, instead of inclining the container holding means 103 by the container inclining means 104, the container inclining means 104 may be configured as nozzle inclining means that enables the nozzle inserting means 106 or the nozzle holding means 105 to rotate with respect to the back plate 122.

In this case, the nozzle inclining means rotates the suction nozzle N in an opposite direction to the direction in which the centrifugal separation container 2 is inclined by the inclining operation means 107 so that the boundary portion 2c is at the lowermost end position, whereby the suction nozzle N is inclined relatively to the centrifugal separation container 2, and the tip end of the suction nozzle N is positioned at the boundary portion 2c that is the lowermost end.

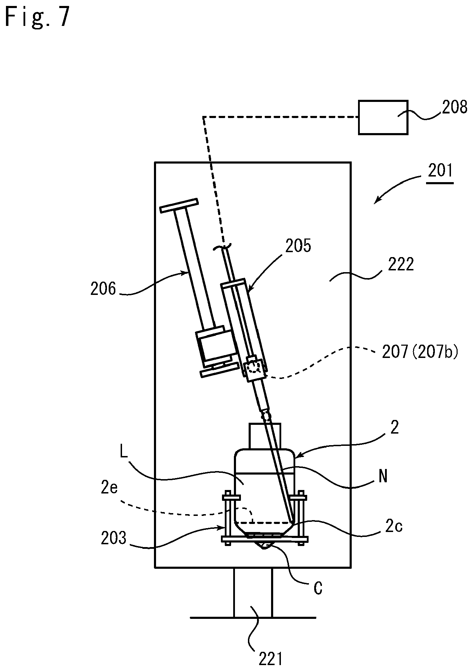

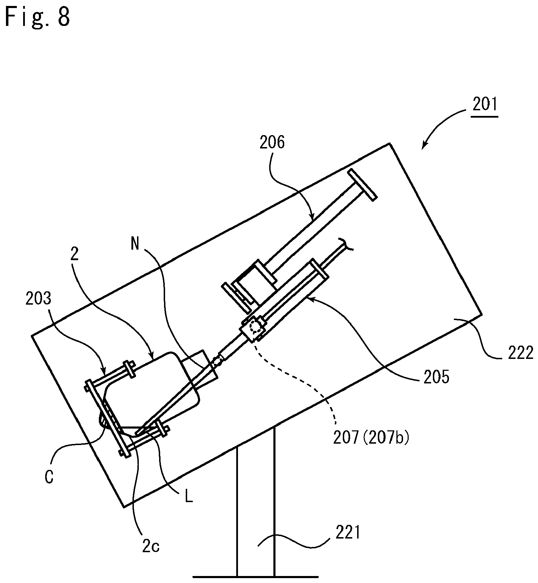

FIG. 7 and FIG. 8 show a liquid suction device 201 according to a third embodiment. Note that in the following explanation, detailed explanation of the same components as those of the above-described second embodiment will be omitted, and numbers that are obtained by further adding 100 to the reference signs used in the second embodiment will be assigned to them.

In the above-described second embodiment, the container inclining means 104 and the nozzle inserting means 106 are integrally inclined by the inclining operation means 107, and thereafter, the container holding means 103 and the centrifugal separation container 2 are inclined by the container inclining means 104, and the tip end of the suction nozzle N is positioned at the boundary portion 2c of the centrifugal separation container 2.

In contrast to this, in the present embodiment, nozzle inserting means 206 is provided on a back plate 222 by being inclined in an opposite direction to a direction in which a centrifugal separation container 2 is to be inclined, with respect to container holding means 203, and the container holding means 203 and the nozzle inserting means 206 are configured to be set to different inclination angles in advance to be integrally inclined by inclining operation means 207.

The inclining operation means 207 is configured by a motor 207a provided at an upper portion of a strut 221, and the back plate 222 is provided at a driving shaft of the motor (not shown).

On the back plate 222 of the inclining operation means 207, the nozzle inserting means 206 is attached with the inclination angle adjusted to a different inclination angle from the inclination angle of the container holding means 203, that is, by being inclined in an opposite direction to a direction in which the container holding means 203 is to be inclined by the inclining operation means 207 with respect to the upright centrifugal separation container 2, and the suction nozzle N is held in an inclined state via the nozzle inserting means 206, as shown in FIG. 7

A position and an angle at which the suction nozzle N is held are set so that a tip end of the suction nozzle N is positioned at a boundary portion 2c between a main housing portion 2a and a bottom housing portion 2b when the suction nozzle N is inserted into the centrifugal separation container 2 by the nozzle inserting means 206. Note that unlike the second embodiment, the container holding means 203 cannot be inclined with respect to the back plate 222.

An operation of the liquid suction device 201 according to the third embodiment having the above-described configuration will be described. The centrifugal separation container 2 housing cells C and a supernatant L is held by the container holding means 203, the suction nozzle N is fitted to the nozzle holding means 205, and the suction nozzle N is held by being inclined in the opposite direction to the direction in which the inclining operation means 207 inclines the container holding means 203 and the nozzle inserting means 206, whereas the centrifugal separation container 2 is in the upright state, as shown in Fig. 7.

When the liquid suction device 201 is operated from this state, the nozzle inserting means 206 inserts the suction nozzle N into the centrifugal separation container 2 first. Since the nozzle inserting means 206 is provided by being relatively inclined in advance with respect to the container holding means 203 at this time, the tip end of the suction nozzle N is positioned at the boundary portion 2c between the main housing portion 2a and the bottom housing portion 2b of the centrifugal separation container 2 by only inserting the nozzle N.

The suction means 208 is temporarily operated in this state to draw out the supernatant L up to a vicinity of a lower end 2e of the main housing portion 2a that is at a height position of the boundary portion 2c. In this state, the supernatant L remains above the cells C of the bottom housing portion 2b of the centrifugal separation container 2.

Thereafter, control means not illustrated operates the inclining operation means 207 to incline the container holding means 203 and the nozzle inserting means 206 provided on the back plate 222 integrally in the same direction.

The inclining operation means 207 inclines the container holding means 203 so that the boundary portion 2c of the centrifugal separation container 2 is at a lowermost end position to collect the remaining supernatant L at the boundary portion 2c that is at the lowermost end position, as in the second embodiment.

Since the tip end of the inserted suction nozzle N is positioned at the boundary portion 2c of the inner peripheral surface in the centrifugal separation container 2, if the suction means 208 is operated again in this state, it is possible to draw out substantially the entire amount of the supernatant L in the centrifugal separation container 2 as much as possible, as in the second embodiment.

Note that also in the present third embodiment, the inclining operation means 207 inclines the container holding means 203 so that the boundary portion 2c of the inner peripheral surface of the centrifugal separation container 2 is at the lowermost end position, and is configured as container inclining means in the present invention, as in the above-described second embodiment.

### Reference Signs List

- 1: Liquid suction device
- 2: Centrifugal separation container
- 2a: Main housing portion
- 2b: Bottom housing portion
- 2c: Boundary portion
- 3: Container holding means
- 4: Container inclining means
- 5: Nozzle holding means
- 6: Nozzle inserting means
- 7: Nozzle inclining means
- 8: Suction means
- N: Suction nozzle
- C: Cells
- L: Supernatant

## Claims

1. A liquid suction device comprising a container holding means that holds a centrifugal separation container in which a mouth portion is formed at an upper portion, and a tubular main housing portion and a conical bottom housing portion below the main housing portion are formed inside, with the bottom housing portion facing down; a cylindrical suction nozzle that is inserted into the centrifugal separation container; and a suction means to which the suction nozzle is connected to draw out a liquid in the centrifugal separation container, the liquid suction device being **characterized by** comprising:
the container inclining means that inclines the container holding means; and nozzle inserting means that inserts the suction nozzle into an inside of the centrifugal separation container, and **characterized in that**
the container inclining means inclines the container holding means so that a boundary portion between the main housing portion and the bottom housing portion in an inner peripheral surface of the centrifugal separation container is at a lowermost end position, and the liquid is drawn out by the suction means in a state in which a tip end of the suction nozzle is positioned at the boundary portion at the lowermost end position.

2. The liquid suction device according to claim 1, **characterized by** comprising:
the nozzle inclining means that inclines the suction nozzle, and **characterized in that**
the suction nozzle is relatively inclined with respect to the centrifugal separation container by the container inclining means or the nozzle inclining means, and the tip end of the suction nozzle is positioned at the boundary portion that is at the lowermost end position.

3. The liquid suction device according to claim 1, **characterized in that** the suction means draws out a predetermined amount of liquid before the tip end of the suction nozzle is positioned at the boundary portion that is at the lowermost end position, and thereafter, draws out the remaining liquid in the state in which the tip end of the suction nozzle is positioned at the boundary portion at the lowermost end position.
